# DEMANDE DE BREVET EUROPEEN

(11) **EP 0 621 479 A1**
(43) Date de publication de la demande: **26.10.1994**
(21) Numéro de dépôt: 94870069.5
(22) Date de dépôt: 19.04.1994
(51) Int. Cl.: G01N 33/564, G01N 33/92

(54) **Procédé de détection et/ou de quantification de différentes classes d'immunoglobulines specifiques d'une pathologie comportant une réponse auto-immunitaire**

(30) Priorité: 23.04.1993 BE 9300413
(71) Demandeur: ANDA BIOLOGICALS S.A., F-67067 Strasbourg Cédex (FR)
(72) Inventeur: Maes, Roland, F-67190 Mutzig (FR); Causse, Jean-Etienne, F-65430 Soues (FR); Labrousse, Hossein, F-65100 Ade (FR)
(74) Mandataire: Van Malderen, Michel

(57) **Abrégé**

L'invention concerne un nouveau procédé de détection et/ou de quantification de différentes classes d'immunoglobulines spécifiques d'une pathologie comportant une réponse auto-immune, présente dans un fluide corporel d'un patient, dans lequel on prélève le fluide corporel du patient, on fait réagir les différentes classes d'immunoglobulines présentes dans ce fluide corporel avec un conjugué antigénique spécifique de la pathologie et constitué d'un acide gras et/ou d'un phospholipide, lié(s) à une protéine hydrosoluble, et avec un agent réactionnel marqué spécifique d'une classe d'immunoglobuline et on détecte et/ou quantifie les différentes classes d'immunoglobulines.

L'invention concerne également la trousse de détection et/ou de quantification de ces différentes classes d'immunoglobulines.

## Description

### I. Objet de l'invention

L'invention concerne un nouveau procédé de détection et/ou de quantification de différentes classes d'immunoglobulines spécifiques d'une pathologie comportant une réponse auto-immunitaire, notamment les maladies auto-immunes et/ou les infections entraînant une perturbation du système immunitaire telles que la sclérose en plaques, la sclérose amyotrophique latérale, l'hyperthyroïdie, l'arthrite, la cirrhose, les lupus, les syndromes antiphosphatides (accident thrombotique, dermatite bulleuse,...), les syndromes néphrotiques, les accidents vasculaires cérébraux, la myasthénie, le cancer, etc.

L'invention concerne également une nouvelle trousse de détection et/ou de quantification de ces différentes classes d'immunoglobulines.

### II. Arrière-plan technologique à la base de l'invention

De nombreuses pathologies comportant une réponse auto-immunitaire ont une étiologie incertaine ou inconnue et peuvent avoir une origine multifactorielle. Apparemment, un dérèglement ou une stimulation du système immunitaire des patients constitue un élément important de la progression de ces maladies.

Dans le cas de la sclérose en plaques (SEP), l'évolution de la pathologie est très similaire à celle observée dans d'autres maladies où intervient un composant immunitaire et peut se résumer ainsi:
- rencontre d'un organisme prédisposé avec un facteur potentiellement pathogène tel une bactérie, un virus ou une toxine;
- longue phase de latence;
- apparition des symptômes. Ceux-ci sont variables et reflètent tous une altération du système nerveux;
- disparition ou atténuation des symptômes avec réapparition aggravée, ultérieurement.

L'étiologie de la sclérose en plaques (SEP) est inconnue et il n'existe pas, pour l'instant, de test diagnostique fiable. Apparemment, des processus auto-immuns sont impliqués dans la genèse des lésions histopathologiques mais il n'est pas possible de dire quels sont les antigènes en cause ni de préciser si la dysfonction immunitaire observée est le résultat ou la cause d'un autre mécanisme, par exemple de type viral, bactérien ou toxique.

### III. Etat de la technique

La recherche de marqueurs immunologiques dans la SEP a porté ces dernières années essentiellement sur les constituants antigéniques de la myéline: la protéine basique, la protéine associée à la myéline, la protéolipide et les glycolipides.

### 1. Protéine basique (PMB)

Des anticorps anti-PMB ont été retrouvés chez des malades atteints de sclérose mais ils ne sont pas spécifiques de la maladie. La protéine libre a été retrouvée dans le liquide céphalo-rachidien de malades mais le taux de PMB et celui des anticorps anti-PMB n'est pas corrélé.

### 2. Protéine associée à la myéline (MAG)

Certains chercheurs trouvent une relation entre la production intrathécale d'IgG et celle d'anticorps anti-MAG dans les scléroses en plaques (SEP) tandis que d'autres retrouvent ces anticorps uniquement dans certaines neuropathies périphériques.

### 3. Protéolipides et glycolipides

Dans tous les cas, la présence des anticorps spécifiques est soit observée de façon épisodique chez les malades souffrant de SEP, soit se retrouvent également au cours d'autres atteintes neurologiques tels les accidents vasculaires cérébraux et les traumatismes crâniens ainsi qu'au cours d'autres maladies telles que les lupus érythémateux.

### 4. Phospholipides et acides gras

Les phospholipides et acides gras ne sont normalement pas antigéniques. Le rôle que peuvent jouer les acides gras dans l'étiologie de la SEP a été mis en question depuis vingt ans mais les résultats obtenus sont contradictoires. Les problèmes essentiels résident dans leur absence d'activité antigénique à l'état libre, dans leur faible solubilité en phase aqueuse et dans les interférences que des molécules de structure chimique très voisine peuvent présenter dans des réactions immunologiques. L'utilisation du phosphatidylinositol libre en tant que marqueur immunologique pour le dépistage de neuropathies démyélinisantes est connue par la demande de brevet FR-90 12578, actuellement abandonnée.

Cette molécule peut provoquer dans certaines situations la formation d'auto-anticorps détectables dans des tests où le phosphatidylinositol est utilisé comme haptène de capture.

Cependant, les résultats obtenus apparaissent extrêmement variables dans des analyses d'homogénéité intra-essais. En effet, l'utilisation de cette molécule libre est très difficile en raison de son insolubilité en phase aqueuse, de la difficulté de son application à l'état libre pour la sensibilisation de phases solides et aussi en raison des interférences immunologiques qui peuvent se produire avec les autres phosphoinositides, le phosphatidylinositol cyclique, les autres phospholipides tels la phosphatidyl sérine, la phosphatidyléthanolamine et la phosphatidylcholine.

### 5. Classes d'anticorps recherchés

La demande de brevet FR-90 12578 divulgue également que seuls les anticorps anti-phosphatidylinositol de classe IgG, séparés des autres classes d'immunoglobulines par chromatographie sur Sephadex 200, sont présents.

Les anticorps IgM sont normalement synthétisés seulement au début d'une infection bactérienne ou virale et leur recherche se justifie pour établir une primo-infection ou une infection récente, tel que cela est pratiqué avec la rubéole chez les femmes enceintes. Cette classe d'anticorps ne se retrouve pas et n'est généralement pas recherchée, dans les maladies chroniques, dont la sclérose en plaques fait partie. Dans ces cas, il est universellement assumé que la recherche d'anticorps de classe IgM est vaine. De leur côté, les anticorps IgA sont communément associés à des processus sécrétoires et se retrouvent essentiellement au niveau des muqueuses et des parois intestinales internes. Une participation des anticorps de type IgA n'a pas été, jusqu'à présent, mise en évidence dans les maladies auto-immunes. Il n'y a donc aucune raison de soupçonner leur intérêt dans des maladies chroniques affectant le système nerveux central.

La détection d'anticorps de classe IgG et IgM est mentionnée pour la cardiolipine, qui est un phospholipide mais uniquement parce que les anticorps anti-cardiolipine sont un index de syphilis (EPA 90402160.7, déposé le 26.07.90).

Une séroanalyse globale, où les anticorps détectés sont supposés être de classe IgG, est donc l'approche considérée comme évidente et suffisante pour le diagnostic des neuropathies démyélinisantes, a fortiori lorsqu'elle est techniquement plus aisée.

### IV. Nouvelle formulation d'un modèle physiopathologique

### 1. Hypothèse de travail

L'échec relatif des recherches portant sur le rôle joué par les différents constituants de la myéline dans l'étiologie de la sclérose en plaques conduit à l'élaboration d'une nouvelle hypothèse.

Une formulation nouvelle d'un modèle physiopathologique de la sclérose en plaques considère que le système immunitaire est constamment stimulé par les auto-antigènes. Les auto-anticorps ne sont pas des éléments aberrants produits de façon extraordinaire par un organisme immunologiquement déréglé mais font partie du répertoire immun: les auto-anticorps existent dans tout sérum normal en petites quantités et leur taux s'accroît dans certaines situations pathologiques. Comme les protéines de la myéline ne semblent pas jouer de rôle décisif dans l'étiologie de la sclérose en plaques, on pourrait envisager d'autres agents perturbateurs, capables de provoquer une montée d'auto-anticorps.

Il a été montré que des phospholipides pouvaient être dégradés par peroxydation au moyen d'oxyhémoglobine, avec production de résidus d'acide azèlaïque. Il était dès lors possible de concevoir le processus démyélinisant observé durant la sclérose comme une maladie auto-immune: un agent perturbateur neurotrope d'origine virale, bactérienne ou toxique, agissant sur un terrain génétiquement favorable, activerait localement des enzymes capables de couper la double liaison en C9 que présentent la vaste majorité des acides gras insaturés. Les résidus créés pourraient ensuite acyler des protéines membranaires. De cette façon, la myéline serait acidifiée et désorganisée. Les modifications membranaires entraînées par la peroxydation lipidique conduiraient à la formation de néo-antigènes capables d'induire des réponses immunes spécifiques. Cette réponse pourrait être assez intense pour pouvoir être mesurée dans le sérum et liquides biologiques des malades atteints de sclérose en plaques. Ces réponses immunes augmenteraient la déstabilisation des membranes et provoqueraient un nouveau processus d'acylation, accompagné de l'exposition d'acides gras non-modifiés, normalement inaccessibles parce qu'enfouis à l'intérieur des bicouches lipidiques. Ces acides gras conjugués à la protéine basique de la myéline, maintenant exposés, seraient capables à leur tour de provoquer une réponse immunitaire, ce qui expliquerait le caractère cyclique et aggravant de la maladie.

### 2. Approche suivie

Les acides gras, lipides, phospholipides, gangliosides et autres constituants normaux des organismes vivants supérieurs ne sont normalement pas immunogéniques mais peuvent, dans certaines circonstances, le devenir, notamment lorsque ces composés sont conjugués à d'autres molécules. Ces lipides et acides gras sont donc des haptènes qui peuvent être utilisés tels quels dans des tests de détection d'anticorps spécifiques qui les reconnaissent. Cependant, la préparation de tests diagnostiques où ces haptènes de capture sont accouplés à des membranes de cellules rouges, ou encore à du latex, du charbon actif, des gels de polysaccharides, des gels de polyacrylamide, des surfaces de polystyrène ou autres, est difficile en raison du fait de leur faible solubilité dans des milieux aqueux. Il est possible, et il a été initialement procédé de la sorte (brevet FR-90 12578), de dissoudre ou diluer les produits dans un solvant organique (éthanol, acétone, diméthylformamide, trichloréthylène, etc.) et de sensibiliser des plaques de microtitration par recouvrement des puits des plaques par un solvant organique contenant des acides gras et lipides dissous, suivi de l'évaporation lente du solvant organique.

Comme ces méthodes sont peu fiables, dangereuses et donnent lieu à des résultats d'analyse où la variabilité est intolérable pour l'utilisation courante de ces tests en pratique médicale, cette technique a été abandonnée.

Une autre approche a consisté en la sensibilisation des supports solides par une glycoprotéine (la β2-glycoprotéine I) capable de réaliser un complexe avec des anticorps et certains phospholipides anioniques tels la phosphatidylserine et la cardiolipine mais très peu le phosphatidylinositol, pourtant également de caractère anionique, et encore moins aux phospholipides neutres tels la phosphatidylcholine. Cette première limitation est doublée par la difficulté d'obtention de la glycoprotéine (Measurement of anti-phopholipid antibodies by ELISA using β2-glycoprotein I as an antigen, J. Immunol. Methods, 143, 1992:223-229).

Le couplage de l'acide oléique et de l'acide azèlaïque à des molécules porteuses et l'utilisation de ces produits dans un test de détection d'auto-anticorps ont été déjà décrits (Identification and Characterization of anti-conjugated Azelaic Acid Antibodies in Multiple Sclerosis, J. of Neuroimmunology, 22 (1989), 129-134; Natural seric antifatty acid antibodies in Multiple Sclerosis, Neuroscience Letters, 80 (1987), 235-239).

Cependant, ces publications ne mentionnent pas l'analyse séparée des différentes classes d'anticorps détectés mais effectuent une analyse globale où est déterminée la présence d'anticorps de toutes classes immunoglobulines (IgG, IgM et IgA confondus).

Un conjugué de phosphatidylinositol avec une molécule porteuse et la possibilité de son utilisation dans un test de détection d'immunoglobulines spécifiques pour le phosphatidylinositol n'a pas été elle décrite (brevet FR-90 12578; Antibodies against phosphatidylinositol in Multiple Sclerosis, Current Concepts in Multiple Sclerosis, H. Wietholter et al., Editors, 1991, 97-102, Elsevier Science Publishers, Amsterdam).

### V. Buts de l'invention

La présente invention vise à mettre au point un procédé de détection et/ou de quantification de différentes classes d'immunoglobulines (de préférence des IgG, des IgM et/ou des IgA) spécifiques d'une pathologie comportant une réponse auto-immunitaire, permettant de caractériser les différents stades et de suivre l'évolution de cette pathologie.

Un autre but de la présente invention est d'obtenir une trousse de diagnostic permettant de détecter et/ou de quantifier ces différentes classes d'immunoglobulines.

### VI. Eléments caractéristiques de l'invention

L'invention concerne un nouveau procédé de détection et/ou de quantification de différentes classes d'immunoglobulines spécifiques d'une pathologie comportant une réponse auto-immune, présentes dans un fluide corporel (tel que le liquide céphalo-rachidien ou le sérum) d'un patient, dans lequel on prélève ledit fluide corporel du patient, l'on fait réagir les différentes classes d'immunoglobulines présentes dans le fluide corporel avec
- un conjugué antigénique spécifique de la pathologie et constitué d'un acide gras, de préférence l'acide oléique ou l'acide azèlaïque et/ou d'un phospholipide, de préférence le phosphatidylinositol, lié(s) à une protéine hydrosoluble, et avec
- un agent réactionnel marqué, spécifique d'une classe d'immunoglobuline, et l'on détecte et/ou quantifie les différentes classes d'immunoglobulines.

L'invention concerne également une trousse de diagnostic et/ou de quantification des différentes classes d'immunoglobulines spécifiques d'une pathologie comportant une réponse auto-immune, caractérisée en ce qu'elle comprend un conjugué antigénique spécifique de la pathologie et constitué d'un acide gras, de préférence l'acide oléique ou l'acide azèlaïque et/ou d'un phospholipide, de préférence le phosphatidylinositol, lié(s) à une protéine hydrosoluble, les immunoglobulines dirigées contre ce conjugué antigénique et/ou un agent réactionnel marqué, spécifique d'une classe d'immunoglobulines.

On entend par les termes "pathologie comportant une réponse auto-immune" les maladies auto-immunes et/ou les infections entraînant une perturbation du système immunitaire, telles que la sclérose en plaques, la sclérose amyotrophique latérale, l'hyperthyroïdie, l'arthrite, la cirrhose, les lupus, les syndromes antiphosphatides (accident thrombotique, dermatite bulleuse,...), les syndromes néphrotiques, les accidents vasculaires cérébraux, la myasthénie, le cancer,...

Avantageusement, la protéine du conjugué est choisie parmi le groupe constitué par l'albumine sérique bovine, l'albumine sérique humaine, la thyroglobuline porcine et/ou la polysérine.

Selon une première forme d'exécution préférée de l'invention, l'agent réactionnel est choisi parmi le groupe constitué par la protéine G, la protéine A et/ou un mélange d'entre elles.

Selon une autre forme d'exécution de l'invention, l'agent réactionnel est un anticorps anti-gammaglobulines de préférence choisi parmi le groupe constitué par les anticorps anti-IgG, anti-IgM, anti-IgA et/ou un mélange d'entre eux.

### Brève description des figures

- Les figures 1 à 3 représentent l'analyse sérologique comparée d'une population saine et d'une population atteinte de sclérose en plaques par le procédé selon l'invention. Le marqueur immunologique est l'acide oléique conjugué à la poly-sérine.
- Les figures 4 et 5 représentent l'analyse sérologique IgA et IgM phosphatidylinositol (PI) et acide azèlaïque de malades sans affection grave et sans sclérose en plaques. Les marqueurs sont conjugués à la PBM.
- Les figures 6 et 7 représentent l'analyse sérologique IgA et IgM phosphatidylinositol (PI) et acide azèlaïque de patients souffrant de sclérose en plaques.
- Les figures 8 à 10 représentent les taux d'anticorps de classe IgA, IgM et IgG à différents stades de SEP comparés à des témoins et des malades souffrant d'autres afflictions.

### Description d'une forme d'exécution préférée de l'invention

Le procédé de détection et/ou de quantification d'immunoglobulines selon la présente invention est basé sur l'utilisation d'un conjugué de l'acide azèlaïque, de l'acide oléique et/ou du phosphatidylinositol avec des molécules hydrosolubles telles que l'albumine bovine sérique, l'albumine sérique humaine et la thyroglobuline porcine. Cependant, d'autres protéines et/ou polypeptides aptes à être conjugués à des acides gras et des phospholipides sont concevables pour l'homme de l'art.

Ce processus transforme les haptènes en antigènes et facilite en même temps leur manipulation. Avantageusement on utilise l'albumine bovine ou humaine.

Ces conjugués sont utilisés dans un système ELISA® pour la détection d'auto-anticorps spécifiques chez les patients atteints de la sclérose en plaques. Parmi toutes les molécules testées, trois conjugués se sont révélés particulièrement adéquats pour la mise en évidence d'auto-anticorps spécifiques: le phosphatidylinositol, l'acide oléique et l'acide azèlaïque qui est un résidu aliphatique en C9 résultant de la coupure et de l'hydrolyse des acides gras insaturés les plus répandus tels l'acide oléique, l'acide linoléique et l'acide linolénique.

D'autres acides gras tels que l'acide subérique (un groupement -CH₂ en moins que l'acide azèlaïque) et l'acide sébacique (un groupement -CH₂ de plus que l'acide azèlaïque), qui peuvent également réagir avec des anticorps spécifiques de l'acide azèlaïque peuvent être également utilisés dans le procédé selon l'invention.

La grande variété de molécules lipophiles montrant entre elles des différences structurelles minimes (par exemple un groupement -CH₂ de plus ou de moins dans une chaîne d'acide gras) induit un élément d'incertitude portant sur la spécificité des réactions immunologiques observées. De façon tout-à-fait inattendue, il a été observé que la conjugaison des trois principaux marqueurs lipophiles de la sclérose en plaques à des protéines hydrosolubles confère au produit de conjugaison des propriétés nouvelles: les marqueurs conjugués sont d'une part d'un emploi facile pour des procédés de sensibilisation de phases solides et, d'autre part, reproduisent l'état sous lequel se trouvent les marqueurs dans les tissus nerveux déstabilisés par une agression; ils sont reconnus de façon plus spécifique dans leurs réactions immunologiques avec des anticorps spécifiques naturels. Le résultat de cette observation fortuite est qu'il est possible maintenant d'élaborer des tests de détection immunologique basés sur des marqueurs conjugués où la précision des mesures effectuées est suffisante pour satisfaire des buts diagnostiques et pronostiques courants. En particulier, l'analyse séparée des auto-anticorps de type IgG, IgM et IgA est maintenant possible. Contrairement à l'opinion prévalente que la recherche des anticorps totaux est suffisante dans un but diagnostique et que seuls les auto-anticorps de type IgG sont synthétisés, nous avons mis en évidence la présence d'anticorps de type IgM et de type IgA, tant pour l'acide azèlaïque que pour l'acide oléique et le phosphatidylinositol, et montrons l'importance de leur analyse distincte pour le diagnostic et le pronostic des scléroses démyélinisantes. En fait, l'analyse et le suivi sérologique IgM et IgA des patients est plus important et donne de meilleures indications que le suivi sérologique des auto-anticorps IgG.

Le couplage d'un acide gras à des protéines ou peptides peut se réaliser de plusieurs façons, connues de l'homme de l'art. Les diverses méthodes apparaissent également adéquates. Nous avons appliqué avec des résultats similaires la méthode de condensation par un carbodiimide insoluble en phase aqueuse (le dicyclohexyl-carbodiimide), un carbodiimide soluble en phase aqueuse (le 1-ethyl-3-(3 diméthyl-aminopropyl-carbodiimide) et la formation d'un anhydride mixte par l'iso-butyl-chloroformate. De façon préférentielle, l'activation des trois marqueurs immunologiques principaux (acide oléique, acide azèlaïque et phosphatidylinositol) se réalise en phase organique par l'éthyl chloroformate.

La détection des auto-anticorps IgG, IgM et IgA spécifiques est réalisée par un test ELISA®: les marqueurs conjugués à des molécules porteuses sont dissous dans un tampon carbonate (0,05 M, pH 9,6) et introduits dans les cupules de plaques de microtitration. Après sensibilisation des plaques par les marqueurs conjugués durant 24 heures à 4°C, celles-ci sont lavées puis couvertes durant deux heures à 37°C par les échantillons (sérum, liquide céphalo-rachidien) dilués dans de l'eau physiologique tamponnée contenant 0,05% de tween 20. Après que la réaction immunologique des anticorps spécifiques contenus dans les échantillons soumis à analyse avec les marqueurs fixés sur la phase solide ait eu lieu, les plaques sont lavées avec de l'eau physiologique tamponnée à pH 7,2 pour éliminer tous les éléments non-spécifiques interférents. La révélation des complexes immuns d'anticorps immunologiquement attachés aux marqueurs immobilisés se réalise par l'incubation des alvéoles avec une solution d'agents de liaison capables de se lier aux gammaglobulines présentes dans le complexe immun. Comme agents réactionnels, on peut utiliser des anticorps anti-gammaglobulines d'espèce ou encore des agents dotés d'un spectre de liaison plus large, tels la protéine A ou la protéine G. Ces agents de liaison sont rendus reconnaissables par leur marquage au moyen d'un élément distinctif, tels une molécule fluorescente, un enzyme, un atome radioactif ou une molécule luminescente, qui permet la reconnaissance aisée de la présence de l'agent de liaison fixé sur le complexe immun que l'on désire reconnaître. De façon préférentielle, un anticorps anti-gammaglobulines humaines, marqué à la peroxydase, est utilisé pour la détection des anticorps humains. Après une étape de lavage, la présence de peroxydase est détectée par l'addition d'un substrat de réaction enzymatique et d'une substance qui change de couleur sous l'effet de la réaction (par exemple H₂O₂ ou peroxyde d'urée avec l'orthophénylène diamine ou la tétraméthylbenzidine).

L'invention sera décrite de manière plus détaillée à l'aide des exemples d'exécution suivants en référence aux figures annexées.

### Exemple 1

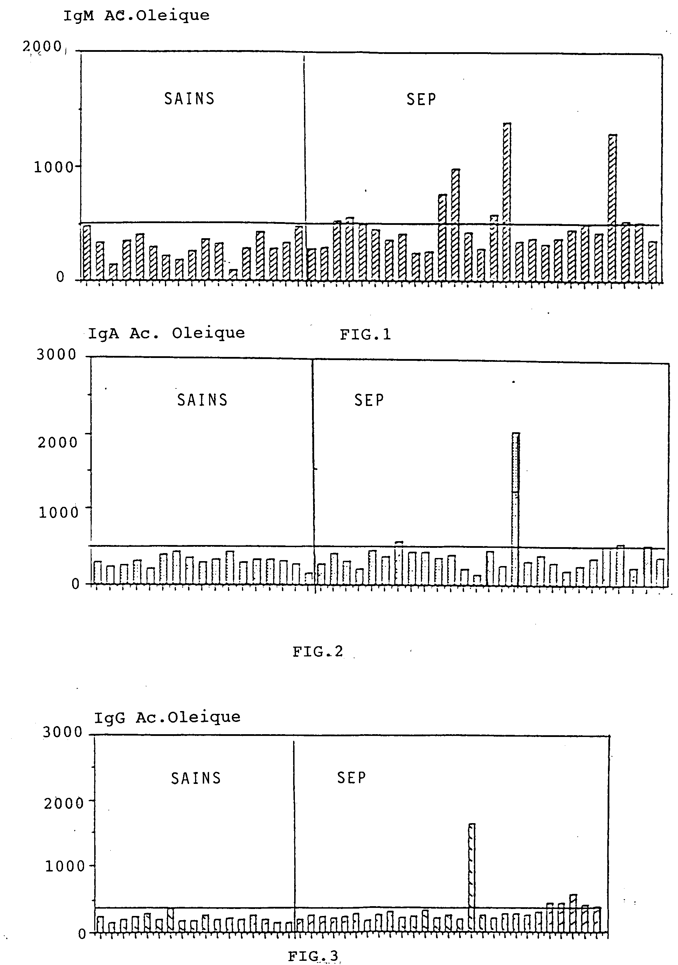

Dans les figures 1 à 3 apparaissent les résultats de l'analyse sérologique effectuée au moyen d'un test ELISA® analysant la présence d'auto-anticorps anti-acide oléique.

Le test est pratiqué de la façon suivante: des cupules de plaques de microtitration (GREINER, NURTLINGEN, Allemagne) ont été sensibilisées par le marqueur immunologique conjugué (acide oléique - thyroglobuline: 5,5 µg/ml, 100 µl/puits). La conjugaison est réalisée selon des techniques déjà connues de l'homme de l'art.

Des sérums dilués (dilution 1:10000) sont incubés dans les cupules durant 2 heures à 37°C, puis les cupules sont lavées. Trois conjugués d'anticorps anti-humains (IgG, dilution 1:20000, IgM et IgA, dilutions 1:10000, dans un tampon phosphate-salin plus 0,05% de tween 20) couplés à de la peroxydase (Dako, Danemark) sont introduits dans les cupules et incubés à 37°C durant 60 minutes. Les cupules sont lavées par de l'eau physiologique puis incubées en présence d'une solution d'eau oxygénée (H₂O₂, 0,03%) et de tétraméthylbenzydine (0,1%) dans un tampon acide citrique-phosphate 0,1 M à pH 5,5. La solution vire au bleu en présence de peroxydase. La réaction est bloquée par addition de 100 µl d'acide sulfurique (0,5 N) après 10 minutes d'incubation à 37°C dans l'obscurité. Les absorbances obtenues par mesure des trois classes d'anticorps (IgG, IgM et IgA) sont données en ordonnées.

Une population de donneurs de sang (17 sujets) est comparée à une population de malades (27 sujets) souffrant de sclérose en plaques.

On voit que le nombre de positifs est nettement plus élevé lorsque l'analyse porte sur la classe IgM d'anticorps, tandis qu'un seul malade conserve la présence d'anticorps anti-acide oléique de classes IgG et IgA.

### Exemple 2

A) Cinq milligrammes de phosphatidylinositol (Sigma, pureté 90%) sont dilués dans 4 ml de diméthylformamide basifiés par 50 µl de triéthylamine, et traités par 50 µl d'éthylchloroformate à 4°C durant 3 minutes.

La suspension de phosphatidylinositol activée est mélangée à 4°C à une solution aqueuse d'albumine bovine délipidée (20 mg/5ml). Le produit de conjugaison est dialysé contre de l'eau physiologique contenant 0,001% de CaCl₂ durant 24 heures à 4°C, puis utilisé pour la sensibilisation de plaques de microtitration (Nunc, Danemark) (2µg/ml dans un tampon carbonate 0,05 M durant 18 heures à 4°C, 250 µl/puits de plaque de microtitration).

B) L'acide azèlaïque a été conjugué à de l'albumine bovine selon le même processus, sauf que le chlorure de calcium a été omis du liquide de dialyse. Les plaques de microtitration ont été sensibilisées par 28µg de conjugué/ml, avec 100 µl/puits.

Une population de 88 sérums est étudiée pour la présence d'auto-anticorps IgM et IgA anti-acide azèlaïque et anti-phosphatidylinositol.

La population analysée se présente comme suit:
1. 36 sérums d'une population hétérogène de malades et de patients hospitalisés sans affection grave déclarée à l'exception d'une cirrhose décompensée.
2. 52 sérums d'une population de patients souffrant de sclérose en plaques. Toutes les phases de la maladie, y compris les cas de rémission, sont inclus dans le groupe.

Les données (absorbances) sont réparties en 3 classes distinctes: une absorbance = 0,75 est considérée comme une valeur-seuil, au-dessus de laquelle les sérums sont positifs, c'est-à-dire possédant un niveau élevé d'anticorps, qui ne se retrouve pas dans les sérums de personnes saines. Entre 0,75 et 0,38 se situe la zone d'incertitude; 0,38 est une deuxième valeur-seuil sous laquelle les valeurs obtenues sont considérées refléter l'absence d'anticorps à un taux significatif.

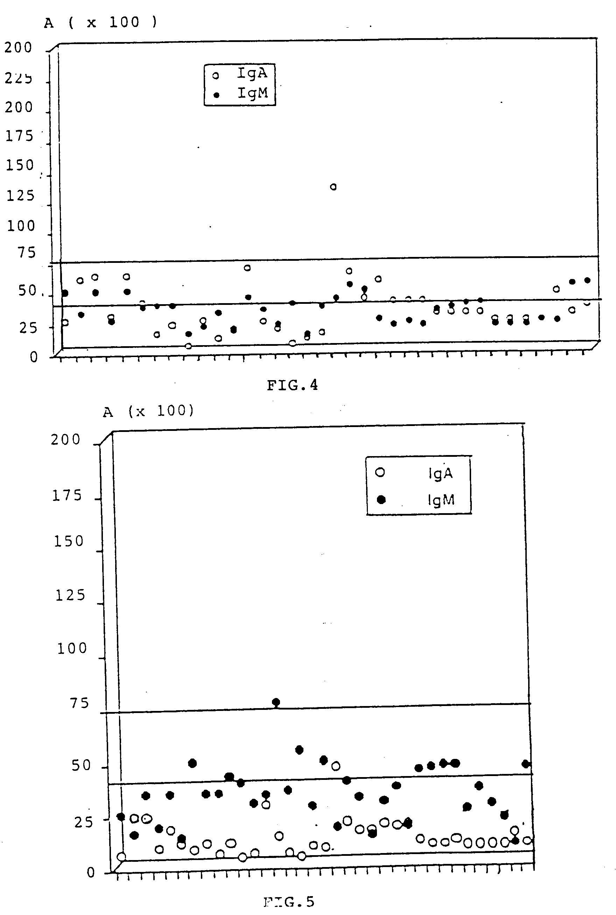

Les figures 4 (phosphatidylinositol) et 5 (acide azèlaïque) montrent les résultats obtenus chez une population "normale" de malades. Un seul malade montre une séropositivité en anticorps anti-phosphatidylinositol IgA. Il s'agit d'un malade souffrant d'une cirrhose décompensée. Un cas marginal d'anticorps anti-acide azèlaïque IgM est également noté, qui est resté sans corrélation clinique.

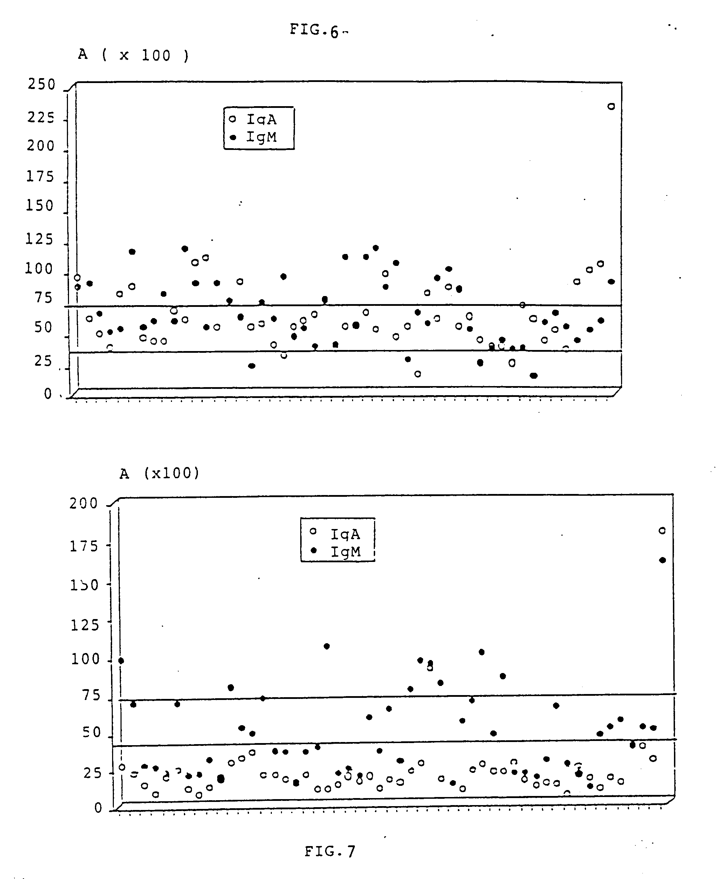

Les figures 6 (PI) et 7 (acide azèlaïque) montrent les résultats obtenus dans l'analyse de malades atteints de sclérose en plaques (toutes phases de la maladie confondues). La fréquence d'anticorps à un titre supérieur à A = 0,75 est élevée dans ce groupe. Les deux seuls cas où des anticorps anti-acide azèlaïque de classe IgA sont détectés sont des formes de poussée invalidante. Ces deux sérums sont également positifs pour les auto-anticorps phosphatidylinositol IgM et IgA. Une telle corrélation ne se retrouve pas pour les autres sérums positifs.

Ayant ainsi établi la possibilité d'utiliser les acide azèlaïque, acide oléique et phosphatidylinositol pour le dépistage de maladies auto-immunes et infections ayant une composante auto-immune par la détection d'auto-anticorps de classe déterminée (IgG, IgM, IgA) on vérifie dans quelle mesure d'autres maladies pouvaient interférer.

### Exemple 3

Une analyse portant sur des SEP cliniquement confirmées, à tous les stades de la maladie, ainsi que l'analyse de témoins et de malades souffrant d'autres afflictions que la sclérose en plaques, a été effectuée selon le protocole expérimental suivant: les sérums dilués ont été analysées pour la présence d'anticorps de classe IgG, IgM et IgA anti-acide oléique, anti-acide azèlaïque et anti-phospatidylinositol. Les mêmes sérums ont été analysés pour la présence de facteurs interférents, en analysant ces sérums dans les mêmes conditions expérimentales en l'absence des 3 haptènes. L'absorbance non-spécifique a été soustraite de l'absorbance spécifique et ramenée à l'unité de l'absorbance non-spécifique selon la formule

### A spécifique - A non-spécifique

### A non-spécifique

Parmi les témoins, un cas à taux élevés d'anticorps est inclus qui, après examen clinique s'est révélé être souffrant de sclérose en plaques. Comme ce cas était initialement inclus parmi les témoins, il n'a pas été changé de catégorie dans les figures.

### A) Anticorps de classe IgA

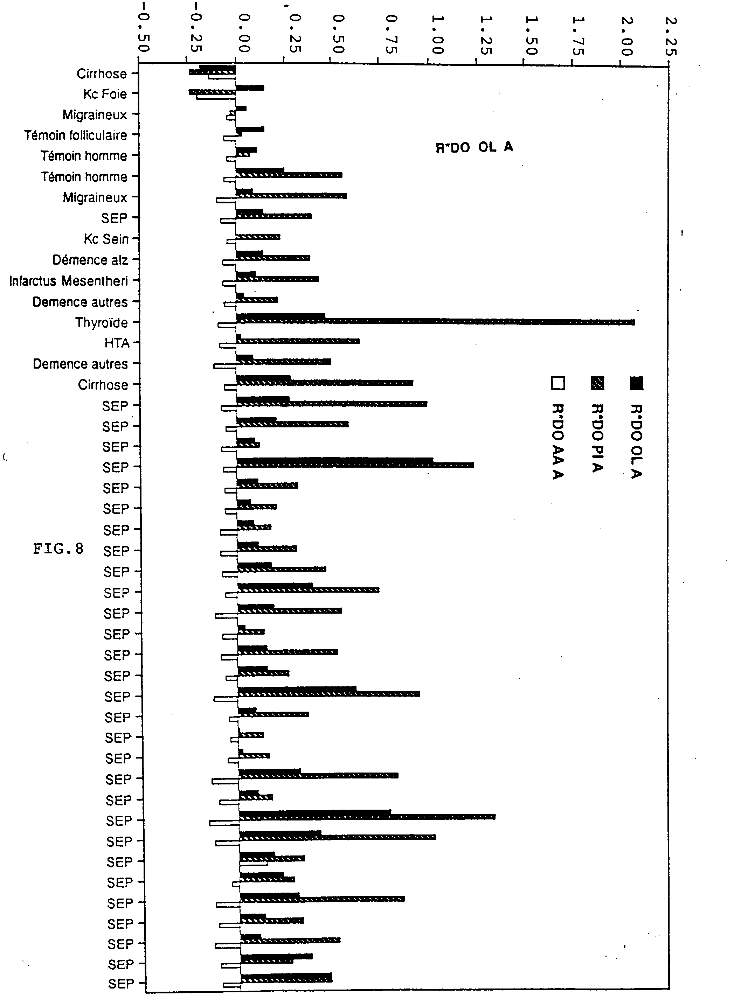

Les taux d'anticorps anti-acide oléique, acide azèlaïque et phosphatidylinositol de classe IgA observés sont rassemblés dans la figure 8. On voit que les anti-PI de classe IgA sont très élevés dans le cas d'hyperthyroïdie et assez élevés dans un cas d'hypertension artérielle (HTA), de même que chez un cirrhotique ainsi que chez un migraineux et un témoin masculin. Un autre témoin à taux élevé a, sur la base de cette élévation, été examiné et trouvé souffrant de sclérose en plaques. Le taux d'anticorps anti-acide oléique est faible chez tous les témoins, excepté le cas d'hyperthyroïdie tandis qu'un taux élevé est assez fréquemment observé dans les cas de SEP. Les anticorps anti-acide azèlaïque de classe IgA sont non-significatifs, sauf dans un cas de SEP.

### B) Anticorps de classe IgM

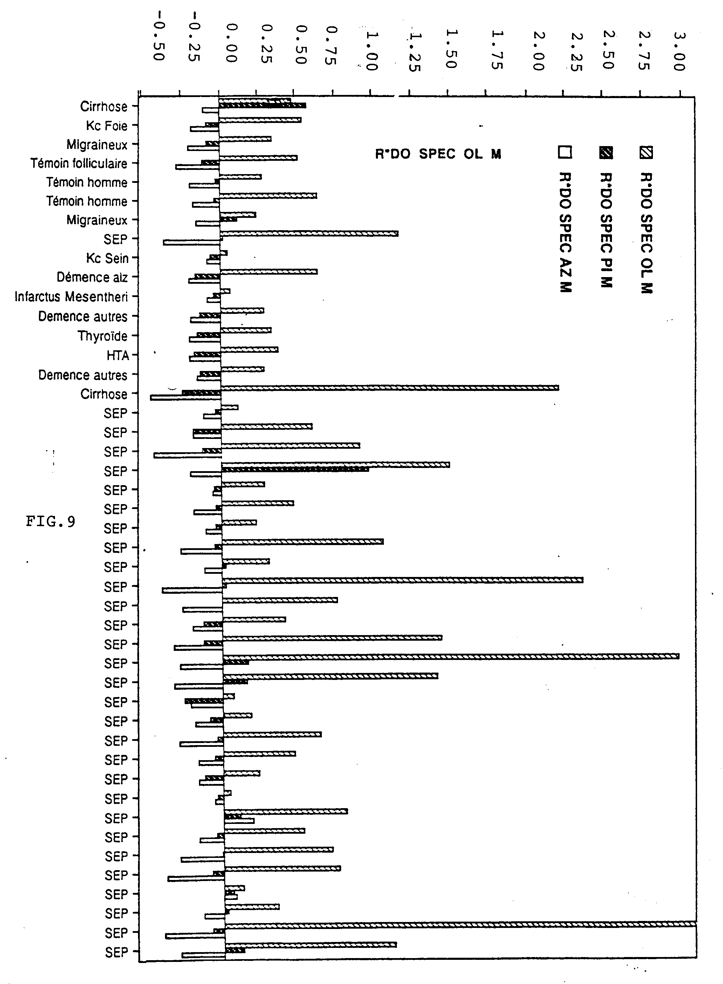

La figure 9 représente les résultats obtenus. Les anticorps de classe IgM anti-acide oléique sont élevés chez un cirrhotique et dans une démence d'Altzheimer, et beaucoup plus fréquents dans les cas de SEP. Le même cas témoin à taux d'anticorps IgA élevés était également positif en IgM.

Les anticorps anti-phosphatidylinositol de classe IgM sont assez élevés chez un cirrhotique et chez un seul cas de SEP clinique. Les anticorps de classe IgM anti-acide azèlaïque ne se retrouvent que dans deux cas de SEP.

### C) Anticorps de classe IgG

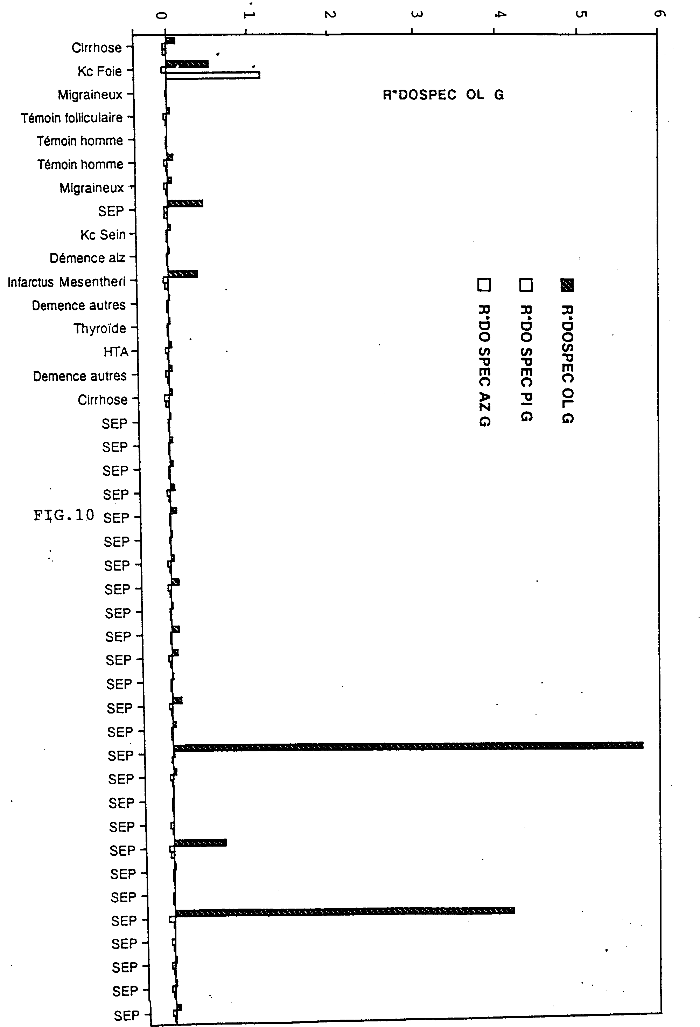

Ceux-ci sont représentés dans la figure 10. On voit qu'ils sont rarement présents.

Cette analyse montre que les trois marqueurs ont, chacun, une signification particulière en fonction de leur fréquence, passant de faiblement à hautement significatif. Ils sont également observés dans d'autres maladies auto-immunes et dans les cancers. Contrairement à ce qui est exposé dans la demande de brevet FR-90 12578, la seule analyse des IgG ou encore une analyse globale des anticorps impliqués dans les réactions analysées ne sont pas suffisants pour établir un diagnostic sérologique complet.

## Revendications

1. Procédé de détection et/ou de quantification de différentes classes d'immunoglobulines spécifiques d'une pathologie comportant une réponse auto-immune, présentes dans un fluide corporel d'un patient, caractérisé en ce que l'on prélève le fluide corporel du patient, en ce qu'on fait réagir les différentes classes d'immunoglobulines présentes dans ce fluide corporel avec un conjugué antigénique spécifique de la pathologie et constitué d'un acide gras et/ou d'un phospholipide, lié(s) à une protéine hydrosoluble, et avec un agent réactionnel marqué spécifique d'une classe d'immunoglobuline et en ce que l'on détecte et/ou quantifie les différentes classes d'immunoglobuline.

2. Procédé selon la revendication 1 caractérisé en ce que l'acide gras du conjugué est l'acide oléique ou l'acide azèlaïque.

3. Procédé selon la revendication 1 caractérisé en ce que le phospholipide du conjugué est le phosphatidylinositol.

4. Procédé selon l'une quelconque des revendications précédentes caractérisé en ce que la protéine du conjugué est choisie parmi le groupe constitué par l'albumine sérique bovine, l'albumine sérique humaine, la thyroglobuline porcine et/ou la poly-sérine.

5. Procédé selon l'une quelconque des revendications précédentes caractérisé en ce que l'agent réactionnel est choisi parmi le groupe constitué par la protéine G, la protéine A et/ou un mélange d'entre elles.

6. Procédé selon l'une quelconque des revendications précédentes caractérisé en ce que l'agent réactionnel est un anticorps anti-gammaglobuline.

7. Procédé selon la revendication 6 caractérisé en ce que l'anticorps anti-gammaglobuline est choisi parmi le groupe constitué par les anticorps anti IgG, anti IgM, anti IgA et/ou un mélange d'entre eux.

8. Trousse de détection et/ou de quantification de différentes classes d'immunoglobulines spécifiques d'une pathologie comportant une réponse auto-immune, caractérisée en ce qu'elle comprend un conjugué antigénique spécifique de la pathologie et constitué d'un acide gras et/ou d'un phospholipide, lié(s) à une protéine hydrosoluble, les immunoglobulines dirigées contre ce conjugué antigénique et/ou un agent réactionnel marqué, spécifique d'une classe d'immunoglobuline.

9. Trousse selon la revendication 8 caractérisée en ce que l'acide gras du conjugué est l'acide oléique ou l'acide azèlaïque.

10. Trousse selon la revendication 8 caractérisée en ce que le phospholipide du conjugué est le phosphatidylinositol.

11. Trousse selon l'une quelconque des revendications 8 à 10 caractérisée en ce que la protéine du conjugué est choisie parmi le groupe constitué par l'albumine sérique bovine, l'albumine sérique humaine, la thyroglobuline porcine et/ou la poly-sérine.

12. Trousse selon l'une quelconque des revendications précédentes 8 à 11 caractérisée en ce que l'agent réactionnel est choisi parmi le groupe constitué par la protéine G, la protéine A et/ou un mélange d'entre elles.

13. Trousse selon l'une quelconque des revendications précédentes 8 à 11 caractérisée en ce que l'agent réactionnel est un anticorps anti-gammaglobuline.

14. Trousse selon la revendication 13 caractérisée en ce que l'anticorps anti-gammaglobuline est choisi parmi le groupe constitué par les anticorps anti IgG, anti IgM, anti IgA et/ou un mélange d'entre eux.
